# EUROPEAN PATENT SPECIFICATION

(11) **EP 1 022 023 B1**
(45) Date of publication and mention of the grant of the patent: **26.10.2005**
(21) Application number: 99204611.0
(22) Date of filing: 30.12.1999
(51) Int. Cl.: A61K 31/195, C12N 1/00, C12N 3/00, A61P 1/00

(54) **Composition suitable as food integrator and for the treatment of intestinal disorders and alterations of the bacterial flora**
Zusammensetzung geeignet als Nahrungsmittelzusatz und zur Anwendung bei Darmerkrankungen sowie bei veränderter Darmflora
Composition pour intégrer dans la nourriture et pour traiter des maladies d'intestin et les altérations de la flore intestinale

(30) Priority: 14.01.1999 IT MI990050
(43) Date of publication of application: 26.07.2000
(73) Proprietor: GIULIANI S.p.A., I-20129 Milano (IT)
(72) Inventor: Bondi, Moreno, 41100 Modena (IT); Frigerio, Giuliano, 20020 Arese (IT); Gatti, Valter, 20135 Milan (IT); Marchioretto, Danila Ingrid, 20099 Sesto San Giovanni (IT); Messi, Paola, 41100 Modena (IT)
(74) Representative: De Gregori, Antonella

(56) References cited:
- EP-A- 0 599 652
- CHATTERJEE, S., ET AL.: "Production of extracellular lysine by a strain of bacillus coagulans. Identification and requirements for growth and lysine accumulation" FOLIA MICROBIOLOGICA, vol. 20, no. 1, 1975, pages 46-51, XP000909479 czechoslovakia
- CHATTERJEE, S., ET AL.: "Effect of antibiotics, gentian violet and tween 80 on growth and lysine accumulation by two species of bacillus" INDIAN J. EXP. BIOL., vol. 14, no. 6, 1976, pages 731-733, XP000909433 india

## Description

Metabolic processes that take place in the intestine are of various nature but fall into two basic categories: metabolic processes that involve hydro-electrolytic interchanges between lumen, tissue fluids and blood, as well as the absorption of terminal fractions of the various primary metabolic processes (glucides, proteids, and lipids), or else the reabsorption of substances relating to the enterohepatic circulation (for example, metabolized bile salts), involving detoxication processes; and, metabolic processes that regard the production of toxic factors and systems responsible for their elimination. In this context, of particular importance on account of their potential toxicity is the production of ammonium radicals (which are extremely harmful for cerebral structures) as terminal process of nitrogenated metabolism and the production of potentially carcinogenic substances, etc.

The intestinal bacterial flora performs a role of enormous importance in the numerous biochemical processes that take place in the intestine, given that the manifold bio-enzymatic activities of this flora bring about important metabolic modifications of the substrates with which they come into contact. It may be useful to recall, in this connection, how the bacterial flora is able to carry out degradation of a number of food substrates that cannot be attacked or digested by the digestive enzymes of the organism. Responsibility for many of the events that are toxic for the organism and responsibility for the phenomena that lie at the basis of ageing is today attributed to the formation of free radicals, a formation that has a ubiquitous character in the various tissues of the organism. However, it would appear to be well ascertained that the formation of free radicals in the structures of the colon is particularly marked also on account of the biological intervention of the intestinal bacterial flora.

A balance between the production of free radicals and anti-oxidant biological mechanisms represents an indispensable condition for maintenance of a state of health. An increase in the production of free radicals ought to be matched by an increase in the processes of detoxication, whereas with ageing there occurs, instead, a reduction in the efficiency of these processes.

Numerous research studies have been dedicated to the development of probiotic products capable of regulating the activity of the gastro-intestinal tract in all cases in which alterations come about in the resident bacterial flora.

However, a number of perplexities have arisen concerning the various biological principles and the preparations containing them as regards their gastroresistance and stability, which are elements that evidently condition the cultural development of the active principle in the intestinal tract and the production of its biological activities.

Indeed, various experimental evidence points to the fact that there occurs a reduction in the microbial content of a product already prior to its expiration and a log loss for limited periods of stay (even just a few hours) in an acid-pH environment, thus underlining how, on the one hand, the shelf-life and, on the other, the acidity of the gastric barrier represent obstacles to an adequate microbial installation at an intestinal level.

The lactoproducing micro-organisms (i.e., those producing lactic acid, L⁺) are the ones most commonly used (it is sufficient to think of milk ferments) in so far as they possess probiotic activity and documented tolerability since frequently they are "orthobiotic" flora, i.e., flora already normally present in the human organism. In view of this fact, according to the purposes of the present invention it is deemed useful to introduce such micro-organisms into man in higher quantities as integrators in order to prepare the organism better for meeting up to specific situations of a physiological nature (intense activity of a physical or intellectual kind, physiological cycles, etc.) or even pathological conditions, i.e., for prophylactic purposes.

The use of lactoproducing biological principles and the production of commercial preparations containing such principles call for a number of basic verifications designed to guarantee optimal growth of the micro-organism and the production of biological activity in the human host.

The basic requisites that are to be ascertained previously are the stability of the biological product in suitable conditions of preservation (possibly at room temperature), resistance to the acidity of the gastric environment, and the capacity to set in progress a regular process of growth of the micro-organism in the intestine, together with all the associated characteristic activities.

In this regard, the present invention proposes a composition that is suitable both as a food integrator and for the treatment of intestinal disorders or alterations of the bacterial flora, characterized in that it contains as active principle a combination of *Bacillus coagulans* registered at the DSHZ collection centre under number DSM 12316 and lysine.

In one first embodiment of the present invention, the said combination is made by associating the aforesaid two components as such.

In an alternative embodiment of the invention, the combination of the active principle is made by first subjecting the *Bacillus coagulans* to a culture in the presence of lysine in the germinative phase, and then bringing it up to the state of spore.

In the latter embodiment of the invention, the *Bacillus coagulans* thus subjected to the culture gives rise to a modified form of the starting bacterium, a modified form that has not so far been described in the existing literature. According to this embodiment of the invention, the active principle hence consists of a new bacterial strain of *Bacillus coagulans* in which the lysine has been incorporated in the growth phase. A culture of this new micro-organism was therefore deposited at the qualified centre for culture collection, DSMZ (Deutsche Sammlung von Mikroorganismen und Zellkulturen GmbH) on July 27, 1998. The registration number of the culture thus filed is DSM 12316 (bacterial strain LAB LYS98).

In a further embodiment of the invention, the active principle is given by the combination of the aforesaid bacterial strain DSM 12316 and again lysine as such. According to the present invention, the bacterial strain of the sporogenous lactobacillus type, *Bacillus coagulans* (otherwise defined in the literature as *Lactobacillus sporogenes),* presents the appropriate requisites in so far as its sporogenous form ensures both stability in the external environment (even at temperatures of the order of 40°C for various months and of the order of 60°C - 90°C for 30 minutes) and resistance to gastric acidity after administration, as well as a high bio-availability in the subsequent portions of the intestine.

The bacterial strain in question is a Gram-positive aerobic and micro-aerophilic bacillus of dimensions ranging between 0.6 - 1 µm and 2.5 - 5 µm, which moves by means of flagella, with a morphology that is typical of *Lactobacilli* (individual bacilli or, more rarely, bacilli arranged in short chains, the length of which varies according to the cultural conditions), and, for this reason is included in the genus *Lactobacillus* in the 5th edition of Bergey's Manual of Determinative Bacteriology. Its taxonomic placing was, however, modified in the 7th edition of Bergey's Manual of Determinative Bacteriology for reasons of simplicity of classification, given that it is able to produce spores which appear under microscopic observation as refractive subterminal/terminal ellipsoidal bodies of dimensions ranging between 0.9 - 1.2 µm and 1.0 - 1.7 µm. Not all authors are, however, in agreement on this form of classification given that there does not appear to be any documented likeness between the two micro-organisms.

According to this criterion, also other catalase-positive aerobic or facultative sporogenous bacilli producing lactic acid are assigned to the genus *Bacillus* in the 8th edition of Bergey's Manual of Determinative Bacteriology. In any case, aside from the taxonomic reference, the name *Bacillus sporogenes* is widely used for this bacterium.

From the biological point of view, *Bacillus coagulans* grows at a temperature of 35-50°C at a pH preferably of between 5.5 and 6.5.

The elective culture medium is "GYE agar medium", where the bacterium develops forming colonies of irregular shape, with lobate margins and umbonate profiles. In the broth formulation itself, growth takes the form of a surface film (area of contact with oxygen), associated with a slight diffused turbidity, whilst in the broth cultures that undergo stirring the growth presents just diffused turbidity.

Among the properties and characteristics that are of importance from the standpoint of use of this biological principle in man, the following are to be emphasized: the capacity to multiply, the capacity to adhere at a cellular level and to produce lactic acid L⁺ in significant quantities as well as bacteriocins (or similar substances) against various bacterial species, including ones not related from the taxonomic viewpoint, among which are included enteropathogenic germs. *Bacillus coagulans* or *Lactobacillus sporogenes* in the vegetative phase shows itself insensitive/resistant to numerous chemobiotics, such as penicillin, cephalosporins, lincomycin, sulphamethizole, trimethoprim-sulphamethoxazole, etc. According to the invention, the aim has been to verify which culture media prove the most suitable for guaranteeing an optimal growth of the cells of *Bacillus coagulans* once they have passed to the vegetative phase, having moreover regard to the choice of principles that prove compatible with human alimentation.

Consequently, microbiological tests have been conducted by adding to the culture medium that is specific for *Lactobacillus* a number of amino acids (among which in particular aspartic acid, lysine, and proline). From these experiments, it surprisingly emerged, according to the invention, that the addition of lysine in certain given concentrations (of the order of 0.1-1 mg/ml) to the medium enhanced the growth of the *Lactobacillus* cells and the formation of lactic acid (L⁺), a phenomenon which does not occur, or occurs only to a decidedly modest extent, with other amino-acid nutrilites. Evaluation of the growth of *Bacillus coagulans* was performed using an agar/Sabouraud's broth to which 0.5% of yeast extract had been added. At this point, an attempt was made to determine which of the various amino-acid components present in the yeast (aspartic acid, proline, lysine, tryptofan), and in what concentrations, were able to favour a more vigorous development of the micro-organism. In this connection, the increase in growth was evaluated at the biophotometer by recording the optical density (A₆₃₀). After a set of preliminary evaluations, two amino acids (lysine and proline) which had yielded the best results were selected and were compared according to the method described in what follows.

### Example A

In special cuvettes for biophotometry, which had been previously sterilized, broth cultures were prepared, which contemplated the addition to the base culture medium (10 ml of Sabouraud's broth) of:

### a) lysine, 0.025 mg/ml; b) proline, 0.01 mg/ml; and c) yeast, 5 mg/ml.

One cuvette containing just Sabouraud's broth was moreover used as control. Each of the four broth cultures was subsequently inoculated with 100 µl of a dilution of a broth culture (overnight incubation) of *Bacillus coagulans* made starting from the lyophilized powder (final concentration of 10⁴ cfu/ml)

The growth of the culture was evaluated in terms of optical density, using a biophotometer, which is an instrument capable of determining the degree of development on the basis of the increase in turbidity. The cuvettes thus prepared were set inside the compartment of the instrument in the presence of a magnetic stirrer that was able to keep the culture in uniform suspension, a condition which enables a proper reading of the turbidity of the culture medium to be made.

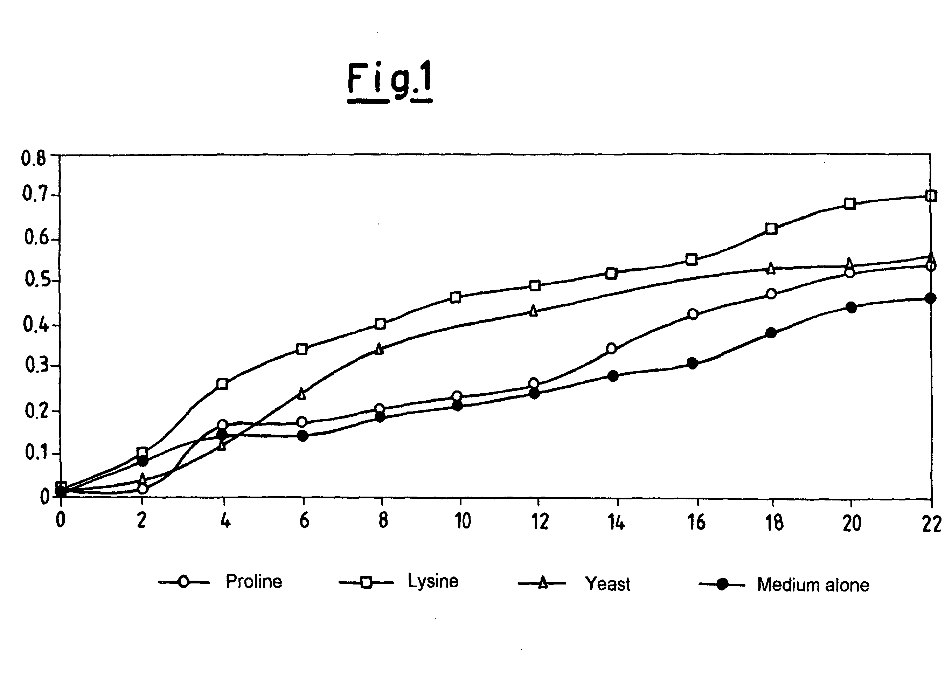

The graph appearing in Figure 1 of the drawings attached to the present description summarizes the results of this test. In this graph the optical density appears on the ordinate, the values being proportional to the number of bacterial individuals produced, whilst the time is given on the abscissa. The four curves shown on the graph identify the behaviour, respectively, of the lysine (used at a concentration of 0.025 mg/ml), yeast (5 mg/ml), and proline (0.01 mg/ml), compared with the Sabouraud's culture medium alone taken as reference standard.

From a comparison of the growth curves obtained after overnight incubation at 37°C, it emerged that among the different substances examined the most effective nutrient was lysine (at a concentration of 0.025 mg/ml).

In fact, the latter amino acid is able to favour an evident increase of the growth curve, reducing the times of the latency phase and increasing the number of cells present at the various times considered. Also the production of lactic acid (L⁺), carried out on a 24-hour broth culture to which lysine was added, proved higher (reaching even a concentration of 486 mg/l) as compared to the blank controls or after the addition of other amino acids.

### Example B

An experiment was conducted, in which *Bacillus coagulans* was grown in culture media containing lysine in three successive stages:
Stage I: growth of the vegetative form in a liquid medium (Sabouraud's broth + lysine 0.25 mg/ml, i.e., at a concentration ten times higher than the one that presented the best yield in the test of Example A);
Stage II: subculture, with overnight incubation at 37°C, in solid culture medium (Sabouraud's agar with addition of the amino acid lysine at the same concentration to enable its further incorporation);
Stage III: further incubation of the plates at room temperature for a time sufficient to cause an evident change in the morphology of the colonies, which, from having an umbonate and mucous appearance, became flat and dry following upon gradual sporulation of the micro-organism.

The micro-organism thus obtained presents characteristics that are new with respect to the starting bacterium. It is a new form in which the lysine has been incorporated by *Bacillus* coagulans in its growth form. This new biological preparation has been registered at DSMZ under the code DSM 12316 (LAB LYS98).

Verification of the increase in growth of the new form DSM 12316 as compared to the starting *Bacillus coagulans* was conducted by evaluating the development of the two strains under examination at the biophotometer according to the procedure described previously.

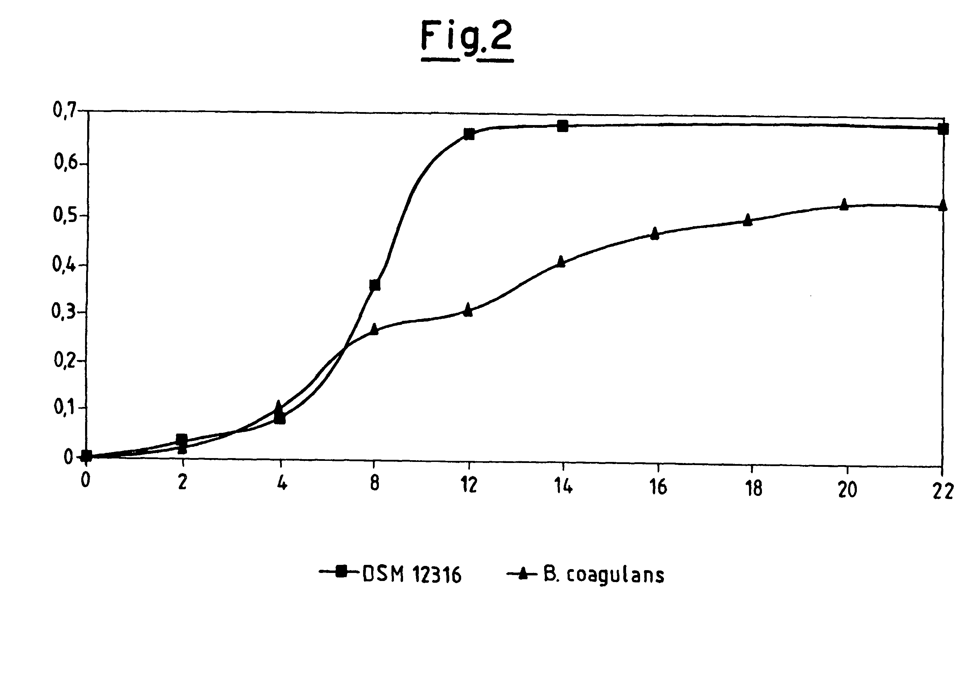

The graph presented in Figure 2 of the attached drawings shows the comparison of the growth curves recorded after overnight incubation at 37°C.

Once again, optical density appears on the ordinate, whilst the time is given in hours on the abscissa. The two curves refer, respectively, as pointed out in the caption of Figure 2, to the growth of *Bacillus* *coagulans* and of DSM 12316 in Sabouraud's broth to which lysine had been added (concentration 0.025 mg/ml).

As may be seen from Figure 2, the growth curve of the new bacterial strain according to the present invention, starting from 8-12 hours, becomes markedly more advantageous in terms of optical density, and hence bacterial growth, as compared to the simple combination of *Bacillus coagulans* and lysine as such. In general, the pharmaceutical or nutritional compositions to which the present invention refers may preferably be of the following types:
a) preparations of *Bacillus coagulans* with the addition of lysine in coated formulations to enable a delayed release of the amino acid in the intestinal tract;
b) preparations of *Bacillus coagulans* pre-cultured in culture media enriched with lysine, in particular preparations of the culture DSM 12316 specified above;
c) preparations comprising the said a) and b) together; and
d) preparations as per points a), b) and c), integrated with other components, among which activated carbon, micronutrients of various types, and natural anti-oxidant principles, such as oleoeuropein or phenylpropanoids of the Ajuga reptans, possibly with appropriate coatings designed to control their release into the gastro-intestinal tract.

The other components according to d) comprise all the vitamins, with particular reference to vitamins with specific anti-oxidant action (vitamins A, C and E) and to vitamins of the B group, various mineral salts or oligo-elements the intake levels of which are recommended on the basis of specific studies (also in this case, contemplating, in particular, the ones linked to mechanisms of detoxication of the accumulation of free radicals, such as copper, zinc, manganese, selenium, magnesium, etc.), the various nucleic fractions and enzymatic principles of natural origin, present, as such or in mixed form, in extractive nutriment.

The range of nutritional principles envisaged must not be considered in any way limiting since it may comprise both compounds/products of an orthomolecular type (i.e., already forming part of the human organism, as is the case of the majority of the substances listed above), and non-orthomolecular products of natural origin, whether vegetable or animal, but in all cases having the character of nutraceutic nutriment, i.e., with a specific scientific connotation in relation to particular metabolic steps.

The non-caloric micronutrients (nutraceutic nutriment) already considered, and probably also others, normally play a role in systemic biochemical processes and perform physiological biochemical functions that can determine the absence of a state of illness and/or persistence of a state of well-being (and hence may not, in actual fact, reveal any visible effect and, as such, may not be perceived by the subject in their biological significance).

According to the invention, compositions can be prepared for the release of the active principles into the distal regions of the intestine to prevent their early absorption.

The "nutriment/vehicle" may comprise any type of foodstuff suitable for not being degraded in the proximal regions of the intestine but upon reaching the colon in an at least partially unmodified form, englobing, in technologically suitable preparations, the micronutritional principles referred to and releasing them prevalently in the colon for them to be better utilized.

An indicative list of macromolecules which may be indigestible or poorly digested by the gastro-intestinal juices, but which are potentially degradable by the enzymatic activity of the intestinal bacterial flora, and ones allowed by current legislation governing foodstuffs is the following: microcrystalline cellulose, methylcellulose, hydroxypropyl cellulose, hydroxypropyl methylcellulose, methylethylcellulose, carboxymethylcellulose, xanthan gum, gum lac, hydrogenated vegetable fats, and pectin. From the standpoint of food technology, different solutions may be envisaged.

One example may be a core of varying shape containing the nutritional substance to be vehicled, generally obtained by means of a standard technology for the manufacture of tablets, coated with a protective layer consisting of adjuvants allowed by current legislation governing foodstuffs, which prevents contact of the core with the gastro-duodenal tract.

For this layer, insoluble or only slightly soluble substances may be used. The desired effect is obtained by means of mechanical erosion, and the thickness of the layer is the factor that determines release of the active principle in the colon or anyway in the distal region of the intestine (gelatinizing substances, gelatine, hydrogenated food-contained fats).

It was also noted that the use of macromolecules having a base of indigestible fibres, prevalently of natural vegetable origin, normally useful for favouring the formation of a voluminous and soft faecal mass, i.e., a physiological faecal mass, surprisingly increased the "nutraceutic" action of a whole series of compounds that fall within the sphere of the essential non-caloric micronutrients when the said nutraceutics were technologically englobed in these macromolecular complexes.

### EXAMPLES OF FORMULATION

For a better understanding of the characteristics of the compositions of the invention, in what follows a number of examples of practical embodiment are given. These examples have a purely illustrative purpose.

In particular, as forms for administration, the following may be cited:
1) hard gelatine capsules containing *Bacillus coagulans* (or the new strain LAB LYS98 as defined above) to the amount of 10-1000 million cfu per capsule, and 10 mg up to 100 mg of lysine in the form of microgranules coated for delayed controlled release;
2) sachets containing powder or granulate;
3) flasks with powder or granulate and a dosing measure; and
4) small drinking flasks.

### EXAMPLE 1 - HARD GELATINE CAPSULES

### Composition

| Description | Quantity | |
|---|---|---|
| *Bacillus coagulans* or LAB LYS98 | 100 (l0-1000*10⁵cfu) | mg |
| L-Lysine hydrochloride (in the gastro-enteroprotected form) | 0-25-250 | mg |
| Magnesium stearate | 4.4 | mg |
| Colloidal silica | 0.8 | mg |
| Dextrose | up to 440 | mg |
| Hard gelatine capsule | 97 | mg |

### EXAMPLE 2 - POWDER IN FLASK WITH DOSING MEASURE

### Composition for 100 g of powder

| Description | Quantity | |
|---|---|---|
| *Bacillus coagulans* or LAB LYS98 | 100 (10-1000*10 ⁵ cfu) | mg |
| L-Lysine hydrochloride (in the gastro-enteroprotected form) | 0-25-250 | mg |
| Vitamin B1 | 1.8 (128% RDA) | mg |
| Vitamin B2 | 2.4 (150% RDA) | mg |
| Vitamin B6 | 3 (150% RDA) | mg |
| Vitamin PP | 27 (150% RDA) | mg |
| Pantothenic acid | 8.28 (138% RDA) | mg |
| Folic acid | 0.3 (150% RDA) | mg |
| Dextrin | 25 | g |
| Dextrose | up to 100 | g |

### EXAMPLE 3 - FLASK WITH DOSING CAP

### Composition of contents of dosing cap

| Description | Quantity | |
|---|---|---|
| *Bacillus coagulans* or LAB LYS98 | 100 (10-1000*10⁵cfu) | mg |
| L-Lysine hydrochloride (in the gastro-enteroprotected form) | 0-25-250 | mg |
| Vitamin B1 | 1.8 (128% RDA) | mg |
| Vitamin B2 | 2.4 (150% RDA) | mg |
| Vitamin B6 | 3 (150% RDA) | mg |
| Vitamin PP | 27 (150% RDA) | mg |
| Pantothenic acid | 8.28 (138% RDA) | mg |
| Folic acid | 0.3 (150% RDA) | mg |
| Maltodextrins | up to 500 | mg |

### Composition of contents of flask

| Description | Quantity | |
|---|---|---|
| *Bacillus coagulans* or LAB LYS98 | 100 (10-1000*10⁵cfu) | mg |
| L-Lysine hydrochloride (in the gastro-enteroprotected form) | 0-25-250 | mg |
| Vitamin B1 | 1.8 (128% RDA) | mg |
| Vitamin B2 | 2.4 (150% RDA) | mg |
| Vitamin B6 | 3 (150% RDA) | mg |
| Vitamin PP | 27 (150% RDA) | mg |
| Pantothenic acid | 8.28 (138% RDA) | mg |
| Folic acid | 0.3 (150% RDA) | mg |
| Maltodextrins | up to 500 | mg |

### EXAMPLE 4 - GRANULATE IN SACHETS

### Composition per sachet

| Description | Quantity | |
|---|---|---|
| *Bacillus coagulans* or LAB LYS98 | 100 (10-1000*10 ⁵ ctu) | mg |
| L-Lysine hydrochloride (in the gastro-enteroprotected form) | 25-250 | mg |
| Vitamin B1 | 1.8 (128% RDA) | mg |
| Vitamin B2 | 2.4 (150% RDA) | mg |
| Vitamin B6 | 3 (150% RDA) | mg |
| Vitamin PP | 27 (150% RDA) | mg |
| Pantothenic acid | 8.28 (138% RDA) | mg |
| Folic acid | 0.3 (150% RDA) | mg |
| Mannitol | 150 | mg |
| Sodium benzosulphimide | 10 | mg |
| Xanthan rubber | 100 | mg |
| Natural flavouring | 100 | mg |
| Sorbitol | up to 3000 | mg |

### EXAMPLE 5 - INDIVIDUAL OR COMBINED CAPSULES

### Composition of capsule with Bacillus coagulans/Lactobacillus sporogenes or LAB LYS98

| Description | Quantity | |
|---|---|---|
| *Bacillus coagulans* or LAB LYS98 | 100 (10-1000*10⁵cfu) | mg |
| L-Lysine hydrochloride (in the gastro-enteroprotected form) | 25-250 | mg |
| Vitamin B1 | 1.8 (128% RDA) | mg |
| Vitamin B2 | 2.4 (150% RDA) | mg |
| Vitamin B6 | 3 (150% RDA) | mg |
| Vitamin PP | 27 (150% RDA) | mg |
| Pantothenic acid | 8.28 (138% RDA) | mg |
| Folic acid | 0.3 (150% RDA) | mg |
| Magnesium stearate | 4.4 | mg |
| Colloidal silica | 0.8 | mg |
| Dextrose | up to 440 | mg |
| Hard gelatine capsule | 97 | mg |

### Composition of capsule with carbon

| Description | Quantity | |
|---|---|---|
| Activated carbon | 150 | mg |
| Hard gelatine capsule in gastroresistant form | 140 | mg |

## Claims

1. Composition suitable both as food integrator and for the treatment of intestinal disorders or alterations of the bacterial flora, **characterized in that** it comprises as active principle *Bacillus coagulans* registered at the DSMZ collection centre under the registration number DSM 12316 and lysines.

2. Composition according to Claim 1, **characterized in that** it is formulated for oral use with excipients for delayed release of the active principle in the intestinal tract.

3. Composition according to Claim 1, **characterized in that** it comprises one or more further ingredients chosen from among vitamins, oligo-elements, mineral salts, anti-oxidants of vegetable origin, such as oleoeuropein and phenylpropanoids, and activated carbon.

4. *Bacillus coagulans* registered at the DSMZ under the registration number DSM 12316.

5. Method for the preparation of a culture of *Bacillus coagulans* incorporating lysine, **characterized in that** it comprises the growth of the vegetative form of Bacillus coagulans in a medium, added with lysine 0.1-1 mg/ml.

6. Method according to claim 5 further comprising the steps of
II) incubating at 37°C overnight, in solid culture medium (Sabouraud's agar) with addition of lysine at the same concentration to enable its further incorporation;
III) further incubation of the plates at room temperature for a time sufficient to cause an evident change in the morphology of the colonies, which, from having an umbonate and mucous appearance, become flat and dry following upon gradual sporulation of the micro-organism.

7. *Bacillus coagulans* registered at the DSMZ collection conter under Registration number DSM 12316 and lysine for use as food integrators or for treatment of intestinal disorders.

## Patentansprüche

1. Zusammensetzung, die sowohl als Nahrungsergänzung als auch zur Behandlung von Darmstörungen oder Veränderungen der Bakterienflora geeignet ist, **dadurch gekennzeichnet, daß** sie als wirksamen Grundstoff *Bacillus coagulans*, registriert beim Kollektionszentrum DSMZ unter der Registernummer DSM 12316, und Lysin umfaßt.

2. Zusammensetzung nach Anspruch 1, **dadurch gekennzeichnet, daß** sie für die orale Anwendung formuliert ist, mit Hilfsstoffen für eine verzögerte Freisetzung des aktiven Grundstoffs im Darmtrakt.

3. Zusammensetzung nach Anspruch 1, **dadurch gekennzeichnet, daß** sie einen oder mehrere weitere Inhaltsstoffe umfaßt, die ausgewählt sind aus Vitaminen, Spurenelementen, Mineralsalzen, Antioxiadantien pflanzlichen Ursprungs wie zum Beispiel Oleoeuropein und Phenylpropanoiden, und Aktivkohle.

4. *Bacillus coagulans*, registriert bei der DSMZ unter der Registernummer DSM 12316.

5. Verfahren zur Herstellung einer Lysin inkorporierenden Kultur von *Bacillus coagulans*, **dadurch gekennzeichnet, daß** es das Wachstum der vegetativen Form von *Bacillus coagulans* in einem Medium, welches mit 0,1 bis 1 mg/ml Lysin versetzt ist, umfaßt.

6. Verfahren nach Anspruch 5, ferner umfassend die Schritte
II) Inkubieren bei 37° C über Nacht in festem Kulturmedium (Sabouraud-Agar) mit Zusatz von Lysin in derselben Konzentration, um dessen weitere Inkorporation zu ermöglichen; und
III) weitere Inkubation der Platten bei Raumtemperatur für eine Zeit, die ausreicht, um eine offensichtliche Änderung der Morphologie der Kolonien zu bewirken, welch letztere, ausgehend von einer knopfartigen und schleimigen Erscheinung, nachfolgend bei der allmählichen Sporulation der Mikroorganismen flach und trocken werden.

7. *Bacillus coagulans*, registriert beim Kollektionszentrum DSMZ unter der Registernummer DSM 12316, und Lysin, zur Verwendung als Nahrungsergänzungen oder zur Behandlung von Darmstörungen.

## Revendications

1. Composition utilisable tant pour la fabrication d'aliments complets que pour le traitement de troubles intestinaux ou d'altérations de la flore bactérienne, **caractérisée en ce qu'**elle comprend, comme principe actif, *Bacillus coagulans*, enregistré auprès du centre de collecte allemand de microorganismes et de cultures cellulaires (DSMZ) sous le numéro d'enregistrement DSM 12316, et de la lysine.

2. Composition selon la revendication 1, **caractérisée en ce qu'**elle est formulée pour une utilisation orale avec des excipients permettant une libération retardée du principe actif dans le tractus intestinal.

3. Composition selon la revendication 1, **caractérisée en ce qu'**elle comprend un ou plusieurs autres ingrédients, choisis parmi les vitamines, les oligo-éléments, les sels minéraux, les anti-oxydants d'origine végétale (comme l'oleuropéine et les phénylpropanoïdes) et le charbon actif.

4. *Bacillus coagulans* enregistré auprès de la DSMZ sous le numéro d'enregistrement DSM 12316.

5. Procédé de préparation d'une culture de *Bacillus coagulans* intégrant de la lysine, **caractérisé en ce qu'**il comprend la culture de la forme végétative de *Bacillus coagulans* dans un milieu additionné de 0,1 à 1 mg/ml de lysine.

6. Procédé selon la revendication 5, comprenant, en outre, les étapes de :
(ii) incubation à 37°C pendant toute une nuit, sur un milieu de culture solide (gélose de Sabouraud) avec adjonction de lysine à la même concentration pour permettre sa future incorporation ;
(iii) incubation supplémentaire des boîtes à température ambiante pendant une durée suffisante pour provoquer une modification évidente de la morphologie des colonies, dont l'apparence ombonée et muqueuse initiale laisse la place à une apparence plate et sèche suite à la sporulation progressive des microorganismes.

7. *Bacillus coagulans* enregistré auprès du centre de collecte allemand DSMZ sous le numéro d'enregistrement DSM 12316 et lysine destinés à être utilisés pour la fabrication d'aliments complets ou pour le traitement de troubles intestinaux.
